# EUROPÄISCHE PATENTSCHRIFT

(11) **EP 0 773 204 B1**
(45) Veröffentlichungstag und Bekanntmachung des Hinweises auf die Patenterteilung: **04.12.2002**
(21) Anmeldenummer: 97101674.6
(22) Anmeldetag: 25.08.1992
(51) Int. Cl.: C07C 13/28, C07F 17/00, C07C 13/465, C07C 49/67

(54) **Verfahren zur Herstellung eines Indens und Verwendung zur Herstellung eines Metallocens**
Process for the preparation of an indene and use thereof for the preparation of metallocenes
Procédé de préparation d'un indène et l'application à la préparation de métallocènes

(30) Priorität: 26.08.1991 DE 4128238
(43) Veröffentlichungstag der Anmeldung: 14.05.1997
(62) Teilanmeldung aus: 92114464.8
(73) Patentinhaber: Basell Polyolefine GmbH, 67056 Ludwigshafen (DE)
(72) Erfinder: Spaleck, Walter, Dr., 65835 Liederbach (DE); Rohrmann, Jürgen, Dr., 65779 Kelkheim (DE); Antberg, Martin, Dr., 65719 Hofheim (DE)

(56) Entgegenhaltungen:
- EP-A- 0 372 414
- EP-A- 0 485 822
- EP-A- 0 485 823
- DE-A- 4 104 931
- FR-A- 1 140 411
- US-A- 3 288 829
- M. ADAMCZYK: "SYNTHESIS OF BIOLOGICAL MARKERS IN FOSSIL FUELS. 2. SYNTHESIS AND C NMR STUDIES OF SUBSTITUTED INDANS AND TETRALINS." J. ORG. CHEM., Bd. 49, Nr. 22, 1984, Seiten 4226-4237, XP002151902
- DATABASE WPI Section Ch, Week 8135 Derwent Publications Ltd., London, GB; Class A60, AN 1981-63033D XP002151904 & JP 56 084731 A (FURUKAWA ELECTRIC CO., LTD.), 10. Juli 1981 (1981-07-10)
- J. E. HORAN: "2-INDANONE" ORGANIC SYNTHESIS, COLL. VOL. V, 1973, Seiten 647-649, XP002151903

## Beschreibung

Die vorliegende Erfindung bezieht sich auf disubstituierte Indene, Verfahren zu ihrer Herstellung und ihre Verwendung zur Herstellung von Metallocenen, die zur Herstellung von Olefinpolymeren mit enger Molmassenverteilung, variabler Molmasse und, im Falle prochiraler Monomere, variabler Mikrostruktur der Kette eingesetzt werden.

Polyolefine mit hoher Molmasse besitzen insbesondere Bedeutung für die Herstellung von Folien, Platten oder Hohlkörpern z.B. Rohre oder Formteile.

Polyolefine mit niedriger Molmasse besetzen Bedeutung für die Herstellung von Additiven oder Gleitmitteln.

In J. Org. Chem. Vol. 49, No. 22, 1984, 4226 bis 4237 wird die Synthese monosubstituierter Indene beschrieben, die zu den entsprechenden monosubstituierten Indanen umgesetzt werden.
In EP-A-0 372 414 werde verbrückte Bisindenylmetallocene beschrieben, die insbesondere am Fünfring des Indenylliganden in der 3-Stellung einen weiteren Substituenten tragen.
In der DE-A-4104931 wird ein Verfahren zur Herstellung von unterschiedlich substituierten Indenen beschrieben.

Aus der Literatur sind lösliche Metallocenverbindungen auf Basis von Bis(cyclopentadienyl)zirkon-alkyl bzw. halogenid in Kombination mit oligomeren Aluminoxanen bekannt. Mit diesen Systemen können Ethylen mit guter und Propylen mit mäßiger Aktivität polymerisiert werden. Man erhält Polyethylen enger Molmassenverteilung und mittlerer Molmasse, das erhaltene Polypropylen ist ataktisch und hat eine sehr niedrige Molmasse.

Die Herstellung von isotaktischem Polypropylen gelingt mit Hilfe des Ethylenbis(4,5,6,7-tetrahydro-1-indenyl)zirkondichlorids zusammen mit einem Aluminoxan in einer Suspensionspolymerisation (vgl. EP-A 185 918). Das Polymer besitzt eine enge Molmassenverteilung. Besonderer Nachteil dieses Verfahrens ist jedoch, daß bei technisch interessanten Polymerisationstemperaturen nur Polymere mit sehr niedriger Molmasse hergestellt werden können.

Es wurde auch eine spezielle Voraktivierungsmethode des Metallocens mit einem Aluminoxan vorgeschlagen, welche zu einer beachtlichen Steigerung der Aktivität des Katalysatorsystems und zu einer deutlichen Verbesserung der Kornmorphologie des Polymeren führt (vgl. DE-OS 37 26 067).

Weiterhin sind Katalysatoren auf Basis Ethylenbisindenylhafniumdichlorid und Ethylen-bis(4,5,6,7-tetrahydro-1-indenyl)hafniumdichlorid und Methylaluminoxan bekannt, mit denen durch Suspensionspolymerisation höhermolekulare Polypropylene hergestellt werden können (vgl. J. Am. Chem. Soc. 109 (1987) 6544). Unter technisch relevanten Polymerisationsbedingungen ist jedoch die Kornmorphologie der derart erzeugten Polymeren nicht befriedigend und die Aktivität der eingesetzten Katalysatoren vergleichsweise gering. Verbunden mit den hohen Katalysatorkosten ist somit mit diesen Systemen eine kostengünstige Polymerisation nicht möglich.

Die letztgenannten Probleme werden im Grundsatz gelöst durch die Verwendung verbrückter Metallocen-Katalysatorsysteme, die in 2-Stellung zur Brücke an beiden aromatischen Liganden eine Alkyl- oder Arylgruppe tragen. Solche Systeme sind in der Z A 91/8925 beschrieben.

Die letztgenannten Katalysatoren weisen in ihren Eigenschaften oder Eigenschaftskombinationen aber noch gewisse Defizite auf, wenn man eine besonders breite Anwendbarkeit für verschiedene Polymerisationsaufgaben und eine technisch und ökonomisch günstige Vorgehensweise in Betracht zieht. Insbesondere ist es erwünscht,
- bei hoher Polymerisationstemperatur, beispielsweise 70°C, zu polymerisieren, weil dann die Katalysatoraktivität hoch ist und zur Abführung der Polymerisationswärme weniger Kühlmedium erforderlich ist als bei niedriger Polymerisationstemperatur,
- bei dieser hohen Polymerisationstemperatur Polyolefine verschieden hoher Molmassen erzeugen zu können, ohne daß Wasserstoff als Molmassenregler benutzt werden muß (die so erzeugten Polymere weisen ungesättigte Endgruppen auf, die für chemische Funktionalisierungen benutzt werden können)
   bei dieser hohen Polymerisationstemperatur in der stereospezifischen Polymerisation unterschiedliche stereotaktische Sequenzlängen erzeugen zu können, die sich beispielsweise bei isotaktischem Polypropylen in unterschiedlichen Schmelzpunkten und weiteren Eigenschaftsunterschieden auswirken
   eine Morphologie des Polymerpulvers mit durchschnittlichen Korngrößen > 1000 µm zu erhalten, da solche Pulver ohne Granulierung direkt auf Verarbeitungsmaschinen beaufschlagt werden können.

Es wurde nun gefunden, daß diese Aufgaben unter Verwendung von mit speziell disubstituierten Indenen hergestellten, verbrückten Metallocenen gelöst werden können.

Die Erfindung betrifft somit Indene der Formel (I) oder (II) 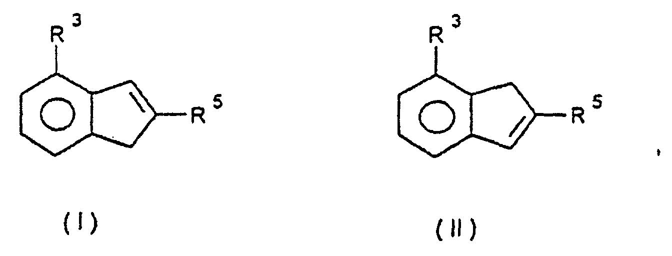 worin
- R³: ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰ oder -PR₂¹⁰-Rest bedeutet, worin R¹⁰ ein Halogenatom, eine C₁-C₁₀-Alkylgruppe oder eine C₆-C₁₀-Arylgruppe ist, und
- R⁵: die für R³ genannte Bedeutung hat.

Weiterhin betrifft die Erfindung die Verwendung eines Indens der Formel (I) oder (II) zur Herstellung von Metallocenen der Formel (III) 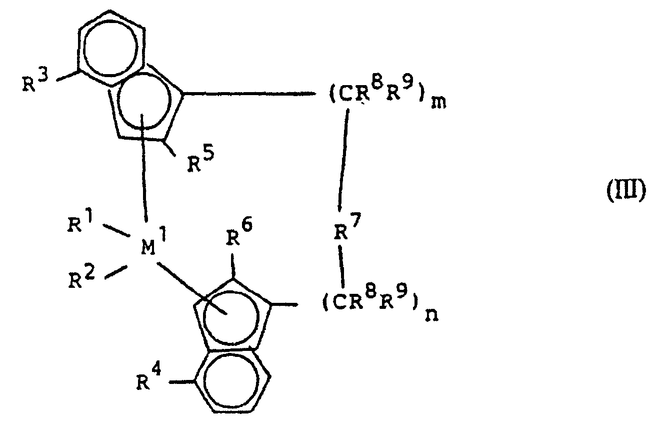 worin
- M¹: ein Metall der Gruppe IVb, Vb oder Vlb des Periodensystems ist,
- R¹ und R²: gleich oder verschieden sind und Wasserstoff, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe; eine C₇-C₄₀-Alkylarylgruppe, eine C₈-C₄₀-Arylalkenylgruppe oder ein Halogenatom bedeuten,
- R³ und R⁴: gleich oder verschieden sind und ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰, SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
- R⁵ und R⁶: gleich oder verschieden sind und die für R³ und R⁴ genannte Bedeutung haben,
- R⁷: 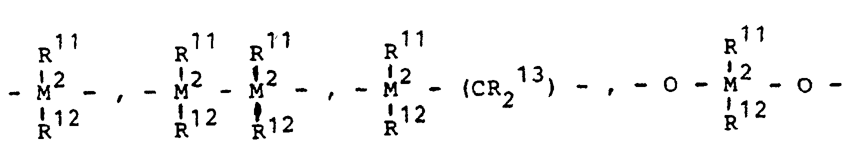 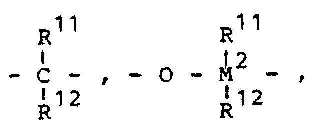
=BR¹¹, =AlR¹¹, -Ge-, -Sn-, -O-, -S-, =SO, =SO₂, =NR¹¹, =CO, =PR¹¹ oder =P(O)R¹¹ ist,
wobei
- R¹¹, R¹² und R¹³: gleich oder verschieden sind und ein Wasserstoffatom, ein Halogenatom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden, oder R¹¹ oder R¹² mit R⁸ oder R⁹ jeweils zusammen mit den sie verbindenden Atomen einen Ring bilden,
M² Silizium, Germanium oder Zinn ist,
R⁸ und R⁹ gleich oder verschieden sind und die für R¹¹ genannte Bedeutung haben und
m und n gleich oder verschieden sind und null, 1 oder 2 sind, wobei m plus n null, 1 oder 2 ist. Alkyl steht für geradkettiges oder verzweigtes Alkyl. Halogen (halogeniert) bedeutet Fluor, Chlor, Brom oder Jod, bevorzugt Fluor oder Chlor.

Der für das Polymerisationsverfahren zu verwendende Katalysator besteht aus einem Cokatalysator und einem Metallocen der Formel (III).

Somit sind die besonders bevorzugten mit den erfindungsgemäßen Indenen der Formel (I) oder (II) hergestellten Metallocene die Verbindungen der Formeln A, B und C 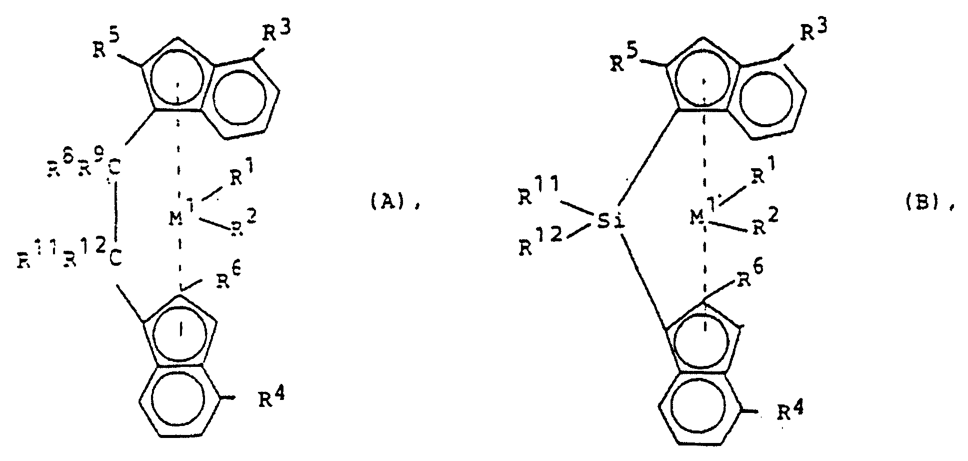 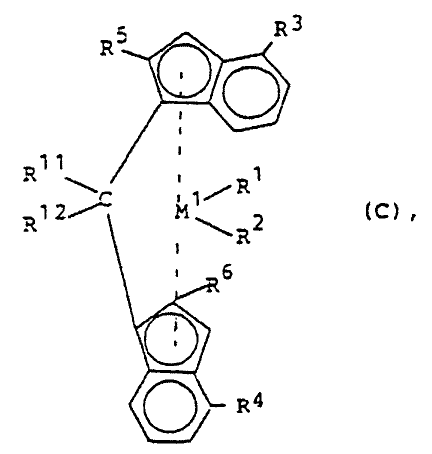 mit
M¹ = Zr, R¹, R²= Methyl, Chlor; R³, R⁴= Methyl, Ethyl, n-Propyl, i-Propyl, n-Butyl, i-Butyl, tert.-Butyl, Neopentyl; R⁵, R⁶= Methyl, Ethyl und R⁸, R⁹, R¹¹, R¹² mit den obengenannten Bedeutungen, insbesondere die in den Beispielen genannten Verbindungen (III).

Zur Herstellung isotaktischer Poly-1-olefine werden die chiralen Metallocene als Racemat eingesetzt.
Verwendet werden kann aber auch die reine R- oder S-Form. Mit diesen reinen stereoisomeren Formen ist optisch aktives Polymeres herstellbar. Abgetrennt werden sollte jedoch die meso-Form der Metallocene, da das polymerisationsaktive Zentrum (das Metallatom) in diesen Verbindungen wegen Spiegelsymmetrie am Zentralmetall nicht mehr chiral ist und daher kein hochisotaktisches Polymeres erzeugen kann. Wird die meso-Form nicht abgetrennt, entsteht neben isotaktischen Polymeren auch ataktisches Polymer. Für bestimmte Anwendungen - weiche Formkörper beispielsweise - kann dies durchaus wünschenswert sein.

Die Trennung der Stereoisomeren ist im Prinzip bekannt.

Die vorstehend beschriebenen Metallocene können nach folgendem Reaktionsschema hergestellt werden:

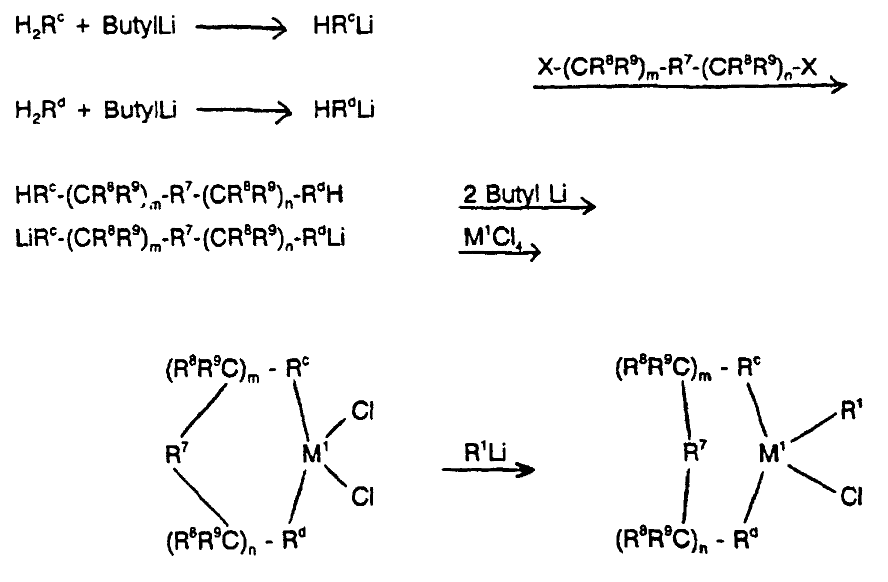

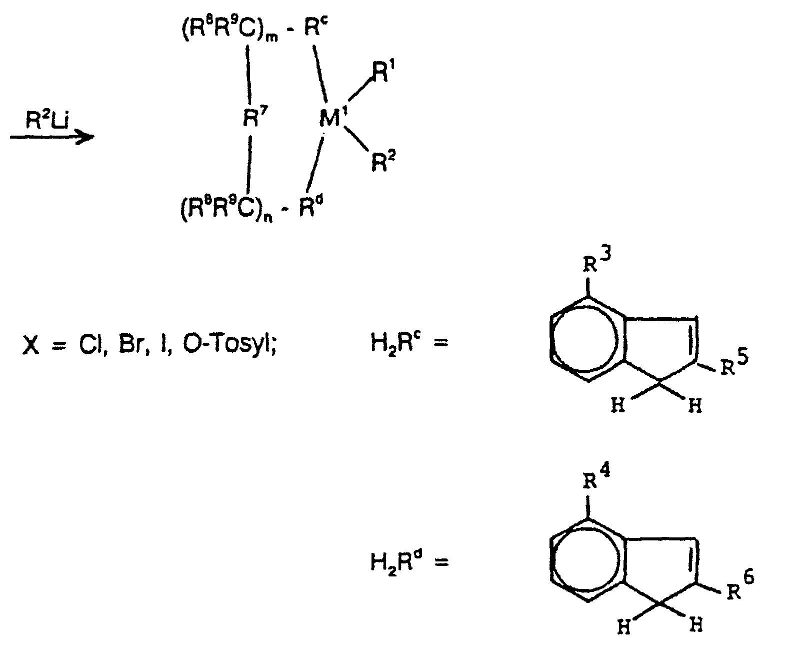

Die Herstellungsverfahren sind literaturbekannt; vgl. Journal of Organometallic Chem. 288 (1985) 63-67, EP-A 320 762 und die Ausführungsbeispiele.

Ein weiterer Gegenstand der vorliegenden Erfindung ist die Herstellung der als Ausgangssubstanzen dienenden erfindungsgemäßen 2,4-substituierten Indene der Formel (I) oder (II) auf 2 verschiedenen Wegen.
a) Als Ausgangsverbindung dient ein Ketoaldehyd der im nachstehenden Reaktionsschema angegebenen Formel, dessen Herstellung bekannt ist (Synthesis 1985, 1058).
Die Umsetzung dieses Ketoaldehyds mit Cyclopentadien wird in einem inerten Lösemittel in Gegenwart einer Base durchgeführt. Bevorzugt werden Alkohole wie Methanol, Ethanol oder t-Butanol, insbesondere Methanol, verwendet.

Als Basen lassen sich eine Vielzahl von Verbindungen verwenden. Als Beispiele seien Alkali- und Erdalkalihydroxide, Alkali- und Erdalkalialkoholate, wie Natriummethanolat, Natriumethanolat, Kaliumtertiärbutanolat, Amide wie Lithiumdiisopropylamid oder Amine genannt. Bevorzugt werden Natriumethanolat, Kaliumtertiärbutanolat und Kaliumhydroxid verwendet.

Die Molverhältnisse der Ausgangsverbindungen einschließlich der verwendeten Base können innerhalb weiter Grenzen schwanken. Bevorzugt ist das Molverhältnis Ketoaldehyd:Cyclopentadien:Base = 1:1-1,5:2-3; insbesondere 1:1,1:2,5.

Die Reaktionstemperatur beträgt bevorzugt -40°C bis 100°C, insbesondere 0°C-25°C.

Die Reaktionszeiten schwanken in der Regel zwischen 10 min und 100 h, vorzugsweise zwischen 1 h und 30 h.

Der Substituent in 2-Stellung kann nach Umwandeln des in 4-Position monosubstituierten Indens in das in 4-Position monosubstituierte 2-Indanon nach einer allgemeinen Arbeitsvorschrift (Organic Synthesis, Coll. Vol. V, 1973, 647) durch eine Grignard-Reaktion eingeführt werden. Die anschließende Wasserabspaltung führt zu den erfindungsgemäßen 2,4-substituierten Indenen.

Die erfindungsgemäßen 2,4-substituierten Indene fallen als Doppelbindungsisomere an, die direkt für die Synthese der entsprechenden Metallocenkomplexe eingesetzt werden können. 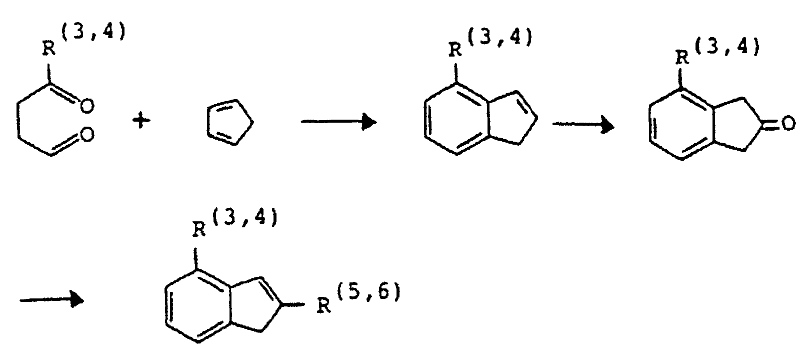 b) Eine andere mögliche und vorteilhafte Strategie verfährt nach folgendem Schema:

Ein in 2-Position substituiertes Benzylhalogenid wird analog eines literaturbekannten Verfahrens (J. Org. chem. 1958, 23, 1437) durch Umsetzen mit einem entsprechend substituierten Malonsäurediester in den disubstituierten Malonsäurediester umgewandelt.

Verseifung des Diesters und Decarboxylierung nach den üblichen Verfahren führt zu einem disubstituierten Propionsäurederivat.

Der Ringschluß zum 2,4-disubstituierten 1-Indanon wird nach Überführen der Carbonsäure in das Carbonsäurechlorid nach gängigen Verfahren durchgeführt.

Reduktion des Ketons nach bekannten Methoden und anschließende Wasserabspaltung liefert die erfindungsgemäßen 2,4-disubstituierten Indene.

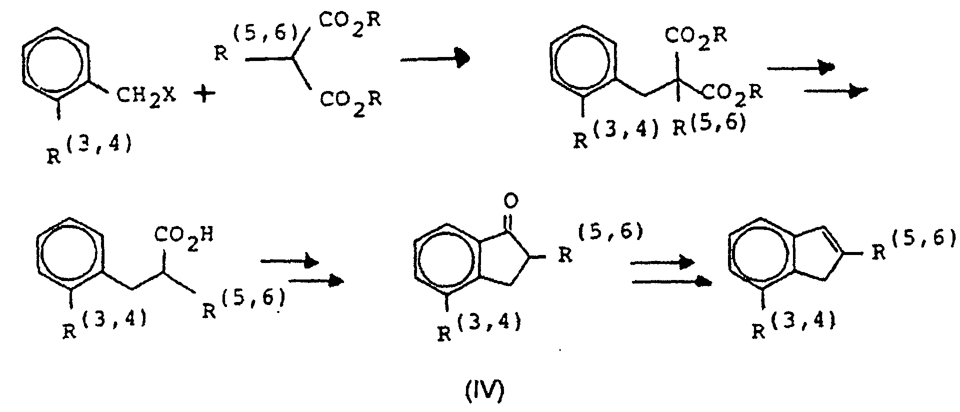

Ein weiterer Gegenstand der Erfindung sind Indanone der Formel (IV) 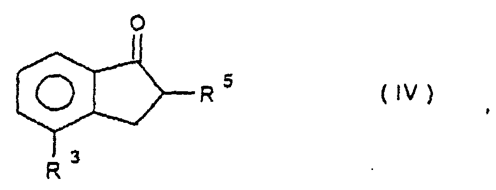 worin
- R³: ein Hatogenatom, eine C₁-C₁₀-Alkylgruppe. die halogeniert sein kann, eine C₆-C₁₀-Arylgruppe, einen -NR₂¹⁰, -SR¹⁰, -OSiR₃¹⁰ oder -PR₂¹⁰-Rest bedeutet, worin R¹⁰ ein Halogenatom, eine C₁-C₁₀-Alkylgruppe oder eine C₆-C₁₀-Arylgruppe ist, und
- R⁵: die für R³ genannte Bedeutung hat,
die zur Herstellung der erfindungsgemäßen Indene der Formel (I) oder (II) dienen.

Als Cokatalysator kann ein Aluminoxan der Formel (V) 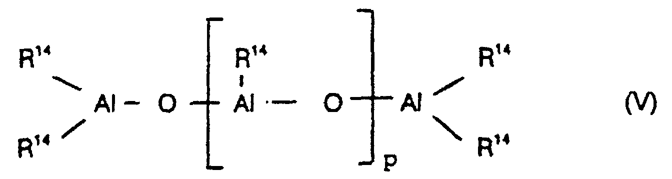 für den linearen Typ und /oder der Formel (VI) 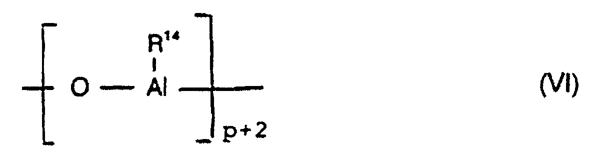 für den cyclischen Typ verwendet werden, wobei in der Formel (V) und (VI) die Reste R¹⁴ gleich oder verschieden sein können und eine C₁-C₆-Alkylgruppe, eine C₆-C₁₈-Arylgruppe oder Wasserstoff bedeuten, und p eine ganze Zahl von 2 bis 50, bevorzugt 10 bis 35 bedeutet.

Bevorzugt sind die Reste R¹⁴ gleich und bedeuten Methyl, Isobutyl, Phenyl oder Benzyl, besonders bevorzugt Methyl.

Sind die Reste R¹⁴ unterschiedlich, so sind sie bevorzugt Methyl und Wasserstoff oder alternativ Methyl und Isobutyl, wobei Wasserstoff bzw. Isobutyl bevorzugt zu 0,01-40 % (Zahl der Reste R¹⁴) enthalten sind.

Das Aluminoxan kann auf verschiedene Arten nach bekannten Verfahren hergestellt werden. Eine der Methoden ist beispielsweise daß eine Aluminiumkohlenwasserstoffverbindung und/oder eine Hydridoaluminiumkohlenwasserstoffverbindung mit Wasser (gasförmig, fest, flüssig oder gebunden - beispielsweise als Kristallwasser) in einem inerten Lösungsmittel (wie z.B. Toluol) umgesetzt wird. Zur Herstellung eines Aluminoxans mit verschiedenen Alkylgruppen R¹⁴ werden entsprechend der gewünschten Zusammensetzung zwei verschiedene Aluminiumtrialkyle (AlR₃ + AlR'₃) mit Wasser umgesetzt (vgl. S. Pasynkiewicz, Polyhedron 9 (1990) 429 und EP-A 302 424).

Die genaue Struktur der Aluminoxane (V) und (VI) ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, das Metallocen vor dem Einsatz in der Polymerisationsreaktion mit einem Aluminoxan der Formel (V) und/oder (VI) vorzuaktivieren. Dadurch wird die Polymerisationsaktivität deutlich erhöht und die Kornmorphologie verbessert.

Die Voraktivierung der Übergangsmetallverbindung wird in Lösung vorgenommen. Bevorzugt wird dabei das Metallocen in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösung. Das Metallocen kann in der gleichen Konzentration eingesetzt werden, vorzugsweise wird es jedoch in einer Menge von 10⁻⁴ - 1 mol pro mol Aluminoxan eingesetzt. Die Voraktivierungszeit beträgt 5 Minuten bis 60 Stunden, vorzugsweise 5 bis 60 Minuten. Man arbeitet bei einer Temperatur von -78°C bis 100°C, vorzugsweise 0 bis 70°C.

Das Metallocen kann auch vorpolymerisiert oder auf einen Träger aufgebracht werden. Zur Vorpolymerisation wird bevorzugt das (oder eines der) in der Polymerisation eingesetzte(n) Olefin(e) verwendet.

Geeignete Träger sind beispielsweise Silikagele, Aluminiumoxide, festes Aluminoxan oder andere anorganische Trägermaterialien. Ein geeignetes Trägermaterial ist auch ein Polymerpulver in feinverteilter Form.

An Stelle oder neben eines Aluminoxans können Verbindungen der Formeln RₓNH₄₋ₓBR'₄, RₓPH₄₋ₓBR'₄, R₃CBR'₄ oder BR'₃ als geeignete Cokatalysatoren verwendet werden. In diesen Formeln bedeutet x eine Zahl von 1-4, bevorzugt 3, die Reste R sind gleich oder verschieden, bevorzugt gleich, und bedeuten C₁-C₁₀-Alkyl, C₆-C₁₈-Aryl oder 2 Reste R bilden zusammen mit dem sie verbindenden Atom einen Ring, und die Reste R' sind gleich oder verschieden, bevorzugt gleich, und stehen für C₆-C₁₈-Aryl, das durch Alkyl, Haloalkyl oder Fluor substituiert sein kann.

Insbesondere steht R für Ethyl, Propyl, Butyl oder Phenyl und R' für Phenyl, Pentafluorphenyl, 3,5-Bistrifluormethylphenyl, Mesityl, Xylyl oder Tolyl (vgl. EP-A 277 003, EP-A 277 004 und EP-A 426 638).

Bei Verwendung der obengenannten Cokatalysatoren besteht der eigentliche (aktive) Polymerisationskatalysator aus dem Reaktionsprodukt von Metallocen und einer der genannten Verbindungen. Daher wird zunächst dieses Reaktionsprodukt bevorzugt außerhalb des Polymerisationsreaktors in einem separaten Schritt unter Verwendung eines geeigneten Lösungsmittels hergestellt.

Prinzipiell ist als Cokatalysator jede Verbindung geeignet, die aufgrund ihrer Lewis-Acidität das neutrale Metallocen in ein Kation überführen und dieses stabilisieren kann ("labile Koordination"). Darüberhinaus soll der Cokatalysator bzw. das aus ihm gebildete Anion keine weiteren Reaktionen mit dem gebildeten Metallocenkation eingehen (vgl. EP-A 427 697).

Zur Entfernung von im Olefin vorhandener Katalysatorgifte ist eine Reinigung mit einem Aluminiumalkyl, beispielsweise AlMe₃ oder AlEt₃ vorteilhaft. Diese Reinigung kann sowohl im Polymerisationssystem selbst erfolgen, oder das Olefin wird vor der Zugabe in das Polymerisationssystem mit der Al-Verbindung in Kontakt gebracht und anschließend wieder abgetrennt.

Die Polymerisation oder Copolymerisation wird in bekannter Weise in Lösung, in Suspension oder in der Gasphase kontinuierlich oder diskontinuierlich, ein- oder mehrstufig bei einer Temperatur von -60 bis 200°C, vorzugsweise 30 bis 80°C, durchgeführt. Polymerisiert oder copolymerisiert werden Olefine der Formel R^{a}-CH=CH-R^{b}. In dieser Formel sind R^{a} und R^{b} gleich oder verschieden und bedeuten ein Wasserstoffatom oder einen Alkylrest mit 1 bis 14 C-Atomen. R^{a} und R^{b} können jedoch auch mit den sie verbindenen C-Atomen einen Ring bilden. Beispiele für solche Olefine sind Ethylen, Propylen, 1-Buten, 1-Hexen, 4-Methyl-1-penten, 1-Octen, Norbornen oder Norbornadien. Insbesondere werden Propylen und Ethylen polymerisiert.

Als Molmassenregler wird, falls erforderlich, Wasserstoff zugegeben. Der Gesamtdruck im Polymerisationssystem beträgt 0,5 bis 100 bar. Bevorzugt ist die Polymerisation in dem technisch besonders interessanten Druckbereich von 5 bis 64 bar.

Dabei wird das Metallocen in einer Konzentration, bezogen auf das Übergangsmetall, von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan wird in einer Konzentration von 10⁻⁵ bis 10⁻¹ mol, vorzugsweise 10⁻⁴ bis 10⁻² mol pro dm³ Lösemittel bzw. pro dm³ Reaktorvolumen verwendet. Die anderen genannten Cokatalysatoren werden in etwa äquimolaren Mengen zum Metallocen verwendet. Prinzipiell sind aber auch höhere Konzentrationen möglich.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Pentan, Hexan, Heptan, Isooctan, Cyclohexan, Methylcyclohexan genannt.

Weiterhin kann eine Benzin- bzw. hydrierte Dieselölfraktion benutzt werden. Brauchbar ist auch Toluol. Bevorzugt wird im flüssigen Monomeren polymerisiert.

Werden inerte Lösemittel verwendet, werden die Monomeren gasförmig oder flüssig zudosiert.

Die Dauer der Polymerisation ist beliebig, da das die erfindungsgemäßen Indene enthaltende Katalysatorsystem einen nur geringen zeitabhängigen Abfall der Polymerisationsaktivität zeigt.

Das Verfahren zeichnet sich dadurch aus, daß die mit den erfindungsgemäßen Indenen hergestellten Metallocen-Katalysatorsysteme im technisch interessanten Temperaturbereich zwischen 30 und 80°C, insbesondere aber im Bereich zwischen 60 und 80°C, Polymere mit enger Molmassenverteilung und grobkörniger Kornmorphologie sowie variabler Molmasse und Stereotaktizität erzeugen. Die jeweils gewünschte Polymermolmasse und Stereotaktizität wird durch Wahl der geeigneten Substituenten in den 2- und 4-Stellungen des Ligandsystems des Metallocens eingestellt. Wird ohne Wasserstoff als Molmassenregler polymerisiert, weisen die Polymeren ungesättigte Endgruppen auf.

Die nachfolgenden Beispiele sollen die Erfindung näher erläutern.

### Beispiele

### I) Metallocen A: rac-Dimethylsilylbis(1-(2-methyl-4-ethylindenyl))zirkondichlorid

### I.1. 4-Ethylinden (a2)

20,7 g (181,7 mmol) 4-Oxocapronaldehyd (a1, hergestellt aus Propionsäurechlorid und Allylchlorid; vgl. Synthesis, (1985) 1058) wurden in 10 ml abs. Methanol gelöst und unter Kühlung mit einer Lösung von 13,2 g (199 mmol) Cyclopentadien in 5 ml abs. Methanol versetzt. Diese Mischung wurde bei 0°C innerhalb 35 min. zu einer Lösung von 51 g (454 mmol) Kaliumtertiärbutylat in 100 ml abs. Methanol getropft, wobei eine Dunkelbraunfärbung auftrat. Nach 2-4 h Rühren bei 0°C und weiteren 2 h bei Raumtemperatur wurde die Mischung auf Eis gegossen, auf pH 6 eingestellt und mit Methylenchlorid extrahiert. Die organische Phase wurde mit gesättigter NaCl-Lösung gewaschen, über Natriumsulfat getrocknet und eingedampft. Das Rohprodukt wurde an 750 g Kieselgel 60 chromatographiert. Mit Hexan/Methylenchlorid (20:1 bis 10:1) ließen sich 11,1 g (43 %) des Indens a2 isolieren (2 Doppelbindungsisomere 3:2).

### 1.2. 4-Ethyl-2-indanon (a3)

33,9 g (235 mmol) 4-Ethylinden (a2) wurden unter Eiskühlung langsam zu einer Mischung von 141 ml Ameisensäure (98-100 %) und 33 ml (340 mmol) H₂O₂ (35 %) getropft (stark exotherme Reaktion). Anschließend wurde noch 2,5 h bei Raumtemperatur gerührt. Die entstehende gelborange Suspension wurde im Wasserstrahlvakuum von überschüssiger Ameisensäure befreit. Das zurückgebliebene gelbe Öl wurde mit 900 ml 2N Schwefelsäure versetzt. Unter Nachdosieren von Wasser wurden insgesamt 3 l Wasser überdestilliert, wobei sich das Produkt als gelbliches Öl in der Vorlage abschied. Das Destillat wurde mit gesättigter Natriumcarbonatlösung neutralisiert und ausgeethert. Die Etherphase wurde über Natriumsulfat getrocknet und eingedampft. Man erhielt 22,4 g (59 %) der Verbindung a3 als weißen Feststoff.

### 1.3. 2-Methyl-4-ethylinden (a4)

Eine Lösung von 22,4 g (140 mmol) a3 in 500 ml Diethylether wurde bei Raumtemperatur unter Ar-Schutz innerhalb 1 h mit 140 ml (420 mmol) einer 3 M etherischen Methylmagnesiumbromid-Lösung versetzt. Anschließend wurde noch 2 h bei Raumtemperatur unter Rückfluß und weitere 15 h bei Raumtemperatur gerührt. Die Mischung wurde auf HCI-saures Eis gegossen und ausgeethert. Nach dem Trocknen über Natriumsulfat wurde das Lösemittel abgezogen. Das zurückgebliebene gelbe Öl(20,3 g) wurde in 800 ml Toluol p.a. aufgenommen, mit 2,2 g (11,5 mmol) p-Toluolsulfonsäurehydrat versetzt und 45 min refluxiert. Nach dem Abkühlen wurde die Lösung mehrmals mit Wasser gewaschen, über Natriumsulfat getrocknet und eingedampft. Der Rückstand wurde an 620 g Kieselgel 60 chromatographiert. Mit Hexan/Methylenchlorid (20:1) ließen sich 5,5 g (25 %) des Indens a4 eluieren (gelbliches Öl). Mit Hexan/Essigester (9:1) konnte noch unverbrauchtes Edukt a3 zurückgewonnen werden.

### I.4. Dimethylsilylbis(2-methyl-4-ethylinden) (a5)

Eine Lösung von 5,5 g (34,8 mmol) a4 in 30 ml THF wurde unter Ar-Schutz bei 0°C langsam mit 14 ml (34,8 mmol) einer 2,5 M n-Butyllithium-Lösung in Hexan versetzt und anschließend 2 h unter Rückfluß erhitzt. Die dunkelbraune Lösung wurde anschließend langsam zu einer Lösung von 2,2 g (17,4 ml) Dimethyldichlorsilan in 15 ml THF getropft. Die Mischung wurde insgesamt 10 h unter Rückfluß erhitzt und über Nacht bei Raumtemperatur gerührt, anschließend wurde auf Eis gegossen und mit Diethylether extrahiert. Der nach Abziehen des Lösemittels verbliebene Rückstand wurde an 200 g Kieselgel chromatographiert. Mit Hexan/Methylenchlorid (20:1 bis 10:1) wurden zunächst 2,0 g unverbrauchtes Edukt a4 eluiert. Anschließend folgten 3,1 g des Produktes a5 (48 % Ausbeute bzgl. Si, 75 % bzgl. umges. Ed.). Die Verbindung fällt als gelbliches Öl an (2 Isomere 3:1).

### I.5. rac-Dimethylsilylbis{1-(2-methyl-4-ethylindenyl)}-zirkondichlorid (A)

Eine Lösung von 3,1 g (8,3 mmol) des Ligandsystems a5 in 30 ml Diethylether wurde bei Raumtemperatur unter Ar-Schutz mit 10 ml (25 mmol) einer 2,5 M Butyllithium-Lösung in Hexan versetzt. Zunächst trat eine Orangefärbung und nach 45 min eine Eintrübung der Lösung ein. Nach dem Rühren über Nacht wurde die nunmehr beigefarbene Suspension mit 10 ml Hexan versetzt und über eine G3-Fritte filtriert. Der Niederschlag wurde mit 20 ml Hexan gewaschen und lange im Ölpumpenvakuum getrocknet. Das nahezu farblose Pulver wurde bei -78°C rasch zu einer Suspension von 1,8 g (7,72 mmol) Zirkontetrachlorid in 30 ml Methylenchlorid gegeben. Die Mischung wurde in 1-2 h auf Raumtemperatur erwärmt und nach 30 min Rühren bei Raumtemperatur vollständig eingedampft. Der im Ölpumpenvakuum getrocknete Rückstand wurde zunächst mit 60 ml Hexan gewaschen. Das Produkt wurde dann durch mehrmaliges Extrahieren mit insgesamt 180 ml Toluol gewonnen. Die vereinigten Extrakte wurden eingeengt und bei -35°C der Kristallisation überlassen. Die 1. Fraktion lieferte 0,76 g des Zirkonocens A in der reinen racemischen Form (orangefarbene Kristalle).Die weiteren Fraktionen enthielten einen steigenden Anteil an der meso-Form. Insgesamt wurden 1,78 g (43 %) der Verbindung A gewonnen. ¹H-NMR (CDCl₃) des Racemats: 6,85-7,55 (m,6,Aromaten-H), 6,80 (s,2,β-H), 2,72 (q,4,CH₂), 2,20 (s,6,CH₃), 1,30 (t,6,CH₃), 1,27 (s.6,Si-CH₃),
¹H-NMR (CDCl₃) der meso-Form: 6,6-7,6 (m,6,Arom.-H), 6,68 (s,2,β-H), 2,7 (q,4,CH₂), 2,48 (s,6,CH₃), 1,13-1,43 (m, 12, Et-CH₃, Si-CH₃).

### II. Metallocen B: rac-Dimethylsilylbis{1-(2-methyl-4-isopropylindenyl)}zirkondichlorid

### II.1 4-Isopropylinden (b2)

Durch Umsetzung von iso-Buttersäurechlorid und Allylchlorid wurde 5-Methyl-4-oxocapronaldehyd (b1) analog zu a1 hergestellt (s. I.1.). 45,6 g (356 mmol) b1 wurden analog Vorschrift I.1. mit Cyclopentadien und Kaliumtertiärbutylat umgesetzt und aufgearbeitet. Die Säulenchromatographie lieferte 19,6 g (35 %) des Indens b2 als gelbes Öl (2 Doppelbindungsisomere).

### II.2. 4-lsopropyl-2-indanon (b3)

33,8 g (213 mmol) der Verbindung b2 wurden analog Vorschrift 1.2. oxidiert und mit Wasser destilliert. Man erhielt 22,6 g (61 %) des Indanons b3 als gelblichen Feststoff.

### II.3. 2-Methyl-4-isopropylinden (b4)

11,1 g (63,8 mmol) des Indanons b3 wurden mit 2,5 eq. Methylmagnesiumbromid analog Vorschrift 1.3. umgesetzt. Die Reaktionszeit betrug 17 h bei Raumtemperatur. Anschließend wurde mit p-Toluolsulfonsäurehydrat 25 min refluxiert. Die Chromatographie lieferte 3,9 g (36 %) des Indens b4 farbloses Öl.

### 11.4. Dimethylsilylbis(2-methyl-4-isopropylinden) (b5)

3,9 g (22,7 mmol) des Indens b4 wurden analog Vorschrift 1.4. mit Dimethyldichlorsilan umgesetzt und aufgearbeitet. Die Säulenchromatographie lieferte neben 0,44 g unverbrauchtem Inden 3,0 g des Produktes b5 als gelbes Öl (Isomere). Die Ausbeute betrug 65 % bzgl. Si und 73 % bzgl. umges. Ed.

### II.5. rac-Dimethylsilylbis{1-(2-methyl-4-isopropylindenyl)}zirkondichlorid (B)

3,0 g des Ligandsystems b5 wurden analog Vorschrift 1.5. deprotoniert und mit 1 eq. Zirkontetrachlorid in 20 ml Methylenchlorid umgesetzt. Nach dem Waschen des Rohproduktes mit 40 ml Hexan wurde das Produkt mit insgesamt 120 ml Toluol extrahiert. Der Toluol-Extrakt wurde im Ölpumpenvakuum eingedampft. Man erhielt 1,7 g (46 %) des Zirkonocens als orangefarbenes Pulver. Das Racemat und die meso- Form lagen im Verhältnis 1:1 vor. Durch Umkristallisation aus wenig Toluol oder aus Toluol/Hexan-Mischungen ließ sich die racemische Form rein isolieren. ¹H-NMR des Racemats (CDCl₃): 6,7-7,5 (m,6,Arom.-H), 6,85 (s,2,β-H),
3,0 (m,2,i-Pr-CH) 2,23 (s,6,CH₃) 1,17-1,37 (d,12,i-Pr-CH₃) 1,27 (s,6,Si-CH₃). ¹H-NMR der meso-Form (CDCl₃): 6,5-7,5 (m,6,Arom.-H) 6,75 (s,2,β-H) 3,0 (m,2,i-Pr-CH) 2,48 (s,6,CH₃) 1,10-1,45 (m,18,i-Pr-CH₃,Si-CH₃).

### III. Metallocen C: rac-Dimethylsilylbis{1-(2-methyl-4-tertiärbutylindenyl)}zirkondichlorid

### III.1. 4-Tertiärbutylinden (c2)

Durch Umsetzung von Pivalinsäurechlorid und Allylchlorid wurde 5,5-Dimethyl-4-oxocapronaldehyd c1 analog zu a1 hergestellt (s. I.1.). 41 g (195 mmol) c1 wurden analog Vorschrift 1.1. mit Cyclopentadien und Kaliumtertiärbutylat umgesetzt und aufgearbeitet. Die Reaktionszeit betrug 19 h bei Raumtemperatur. Die Säulenchromatographie lieferte 3,2 g (10 %) des Indens c2 als gelbes Öl (2 Doppelbindungsisomere).

### III.2. 4-Tertiärbutyl-2-indanon (c3)

8,5 g (49,4 mmol) der Verbindung c2 wurden analog Vorschrift I.2. oxidiert und mit Wasser destilliert. Die Reaktionszeit betrug 4 h bei Raumtemperatur. Man erhielt 2,8 g (30 %) des Indanons c3 in Form gelber Kristalle.

### III.3. 2-Methyl-4-tertiärbutylinden (c4)

3,6 g (19 mmol) des Indanons c3 wurden mit 3,0 eq. Methylmagnesiumbromid analog Vorschrift 1.3. umgesetzt und aufgearbeitet. Die Reaktionszeit betrug 17 h bei Raumtemperatur und weitere 4 h unter Rückfluß. Anschließend wurde mit p-Toluolsulfonsäurehydrat 25 min refluxiert. Die Chromatographie lieferte 1,2 g (33 %) des Indens c4 als gelbes Öl. Mit Hexan/Essigester (9:1) konnte unverbrauchtes Edukt zurückgewonnen werden.

### III.4. Dimethylsilylbis(2-methyl-4-tertiärbutylinden) (c5)

1,2 g (6,4 mmol) des Indens c4 wurden analog Vorschrift I.4. mit Dimethyldichlorsilan umgesetzt und aufgearbeitet. Die Reaktionszeit betrug 10 h bei Rückfluß und 3 Tage bei Raumtemperatur. Die Säulenchromatographie lieferte neben 0,48 g unverbrauchtem Inden c4 0,40 g des Produktes c5 als gelbes Öl (Isomere). Die Ausbeute betrug 29 % bzgl. Si und 49 % bzgl. umgesetztes Edukt c4.

### III.5. rac-Dimethylsilylbis{1-(2-methyl-4-tertiärbutylindenyl)} zirkondichlorid (C)

0,40 g (0,93 mmol) des Ligandsystems c5 wurden in 9 ml Diethylether unter Ar-Schutz mit 0,74 ml (1,86 mmol) einer 2,5 M n-Butyllithium-Lösung in Hexan versetzt. Nach Rühren über Nacht wurde die orange Lösung vollständig eingedampft. Der Rückstand wurde lange im Ölpumpenvakuum getrocknet und rasch bei -78°C zu einer Suspension von 225 mg (0,96 mmol) Zirkontetrachlorid in 5 ml Methylenchlorid gegeben. Die Mischung wurde 2 h bei 0°C und 30 min. bei Raumtemperatur gerührt und vollständig eingedampft. Das Produkt wurde mit insgesamt 8 ml Toluot extrahiert. Nach Abziehen des Toluols erhielt man 210 mg (37 %) des Zirkonocens als oranges Pulver. Das Verhältnis von Racemat zur meso-Form betrug 1:1. Durch Umkristallisation aus Toluol/Hexan konnte die reine racemische Form gewonnen werden. ¹H-NMR des Racemats (CDCl₃): 6,8-7,5 (m,6,Arom.-H) 6,92 (s,2,β-H) 2,27 (s,6,CH₃) 1,22-1,41 (m,24,t-Bu,Si-CH₃). ¹H-NMR der meso-Form (CDCl₃): 6,7-7,6 (m,6,Arom.-H) 6,7 (s,2,β-H) 2,50 (s,6,CH₃) 1,1-1,5 (m,24,t-Bu,Si-CH₃).

### IV. Metallocen D: rac-Methylphenylsilylbis{1-(2-methyl-4-isopropylindenyl)}zirkondichlorid

### IV.1. Methylphenylsilylbis(2-methyl-4-isopropylinden) (d5)

Eine Lösung von 2,0 g (11,8 mmol) 2-Methyl-4-isopropylinden b4 (s. II.3.) in 40 ml THF wurde unter Ar-Schutz bei 0°C mit 4,8 ml einer 2,5 M Butyllithium-Lösung in Hexan versetzt und 90 min zum Rückfluß erhitzt. Anschließend wurde die rote Lösung zu einer Lösung von 1,12 g (5,9 mmol) Methylphenyldichlorsilan in 15 ml THF gegeben und 7 h unter Rückfluß erhitzt. Die Mischung wurde auf Eis gegossen und ausgeethert. Die über Natriumsulfat getrocknete Etherphase wurde im Vakuum eingedampft. Der verbleibende Rückstand wurde an 200 g Kieselgel 60 chromatographiert. Mit einem Laufmittelgemisch aus Hexan/Methylenchlorid (10:1) wurden zunächst 0,57 g unverbrauchtes Inden b4 zurückgewonnen. Mit Hexan/Methylenchlorid (10:2) folgten 1,2 g des Produktes d5. Die Ausbeute betrug 44 % bzgl. Si und 61 % bzgl. umges. Inden b4.

### IV.2. rac-Methylphenylsilylbis{1-(2-methyl-4-isopropylindenyl)}zirkondichlorid (D)

Eine Lösung von 1,28 g (2,76 mmol) des Ligandsystems d5 in 20 ml Diethylether wurde bei Raumtemperatur unter Ar-Schutz langsam mit 3,3 ml (8,3 mmol) einer 2,5 M Butyllithium-Lösung in Hexan versetzt und über Nacht gerührt. Die orangefarbene Lösung wurde vollständig eingedampft, lange im Ölpumpenvakuum getrocknet und mit insgesamt 20 ml Hexan gewaschen. Der Rückstand wurde lange im Ölpumpenvakuum bei 40°C getrocknet und pulverisiert. Das gelbe Pulver wurde bei -78°C zu einer Suspension von 0,62 g (2,66 mmol) Zirkontetrachlorid in 15 ml Methylenchlorid gegeben. Die Mischung wurde in 1 h auf 0°C aufgewärmt und weitere 2 h bei Raumtemperatur gerührt. Die rotbraune Suspension wurde vollständig eingedampft und im Ölpumpenvakuum getrocknet. Mit Toluol wurden 1,05 g (63 %) des Zirkonocens extrahiert (oranges Pulver). Im Rohprodukt lagen 1 racemische und 2 meso-Formen im Verhältnis 2:1:1 vor. Durch Umkristallisation aus Toluol/Hexan konnte die racemische Form isoliert werden.
¹H-NMR der Isomerenmischung (CDCl₃): 6,4-8,2 (m,Arom,-H,β-H) 3,1 (br,i-Pr-CH) 2,55 (s,CH₃) 2,33 (s,CH₃) 2,22 (s,CH₃) 1,95 (s,CH₃) 1,13-1,47 (m,i-Pr-CH₃,Si-CH₃).

### V. Metallocen E: rac-Dimethylsilylbis{1-(2-ethyl-4-methylindenyl)}zirkondichlorid

### V.1. 2-(2-Methylbenzyl)-buttersäure (e1)

14,2 g (0,62 mol) Natrium wurden in 250 ml Ethanol gelöst und 118,4 g (0,63 mol) Ethyl-diethylmalonester zugegeben. Es wurden 118,5 g (0,64 mol) 2-Methylbenzylbromid so zugetropft, daß die Mischung leicht siedete. Anschließend wurde 4 h unter Rückfluß erhitzt. Die Suspension wurde auf Wasser gegeben, mit Ether extrahiert und die vereinigten organischen Phasen wurden über MgSO₄ getrocknet. Das Lösungsmittel wurde i.V. entfernt und das erhaltene Rohprodukt (187 g) ohne weitere Reinigung weiter umgesetzt.

Zur Verseifung wurde in Gegenwart von 139 g KOH in 355 ml Ethanol und 170 ml H₂O 15 h unter Rückfluß erhitzt. Das Lösungsgemisch wurde i.V. abgezogen und der Rückstand unter Kühlung mit konz. Salzsäure bis zum pH 1 versetzt. Es wurde 3mal mit Ether extrahiert, die vereinigten organischen Phasen wurden mit gesättigter wäßriger NaCl-Lösung gewaschen und über MgSO₄ getrocknet. Das Lösungsmittel wurde entfernt und der Rückstand zur Decarboxylierung auf 170°C erhitzt, wobei das Produkt e1 abdestillierte (140-145°C/0.1 Torr).
Ausbeute: 96,0 g (81 %).

### V.2. 2-(2-Methyl-benzyl)-buttersäurechlorid (e2)

96 g (0,5 mol) 2-(o-Xylyl)-buttersäure (e1) wurden langsam mit 89 g (0,75 mol) SOCl₂ erhitzt und bis zum Ende der Gasentwicklung (1 h) refluxiert. Überschüssiges Thionylchlorid wurde abdestilliert, und Reste durch dreimaliges Abziehen von je 50 ml Toluol i.V. entfernt. Das Rohprodukt wurde durch Destillation (103°C/1 Torr) gereinigt.
Ausbeute: 101,7 g (96 %, 0,48 mol).

### V.3. 2-Ethyl-4-methyl-1-indanon (e3)

101,7 g (0,48 mol) 2-(2-Methyl-benzyl)-buttersäurechlorid (e2) wurden zu 191 g (1,43 mol) AlCl₃ in 600 ml Toluol zugetropft und ca. 3,5 h auf 80°C erwärmt. Die Reaktionsmischung wurde auf 1 l Eis/konz. HCI gegossen und die Phasen wurden getrennt. Die wäßrige Phase wurde 4mal mit je 250 ml Toluol extrahiert, die vereinigten organischen Phasen mit gesättigter wäßriger NaHCO₃- und NaCI-Lösung gewaschen und über MgSO₄ getrocknet. Das Lösungsmittel wurde i.V. entfernt und der Rückstand destilliert (78°C/0,2 Torr).
Ausbeute: 81 g (97 %, 0,464 mol).

### V.4. 2-Ethyl-4-methyl-inden (e4)

34.1 g (196 mmol) 2-Ethyl-4-methyl-1-indanon (e3) wurden in 210 ml THF/Methanol (2:1) portionsweise mit 11,1 g (294 mmol) NaBH₄ versetzt und 15 h bei Raumtemperatur gerührt. Die Reaktionsmischung wurde auf Eis gegossen und mit konz. HCI bis pH = 1 versetzt. Nach Extraktion mit Ether wurden die vereinigten organischen Phasen mit gesättigter wäßriger NaHCO₃- und NaCl-Lösung gewaschen und über MgSO₄ getrocknet. Der i.V. vom Lösungsmittel befreite Rückstand (36,2 g) wurde für die folgende Eliminierung direkt weiter umgesetzt.

Das nicht gereinigte 2-Ethyl-4-methyl-1-indanol wurde in 700 ml Toluol in Gegenwart von 0,75 g p-Toluolsulfonsäure-monohydrat 2 h auf dem Dampfbad behandelt. Das Lösungsgemisch wurde i.V. entfernt, der Rückstand in Ether aufgenommen, mit gesättigter NaHCO₃- und NaCl-Lösung gewaschen und über MgSO₄ getrocknet. Das Lösungsmittel wurde i.V. entfernt und der Rückstand destilliert (62°C/0,2 Torr).
Ausbeute: 25,7 g (83 %, 162 mmol).

### V.5. Dimethylsilylbis(2-ethyl-4-methylinden) (e5)

Zu 10,4 g (65,5 mmol) 2-Ethyl-4-methyl-inden (e4) in 50 ml abs. THF wurden 26,2 ml (65,6 mmol) einer 2,5 M BuLi-Lösung in Hexan langsam zugetropft und 2 h bei 50°C weitergerührt. Währenddessen legte man 3,95 ml Me₂SiCl₂ in 50 ml abs. THF vor und tropfte anschließend das Li-Salz im Verlauf von 8 h zu. Man rührte 15 h entfernte das Lösungsmittel i.V., suspendierte in n-Pentan und filtrierte erneut. Nach Entfernen des Lösungsgemisches reinigte man durch Säulenchromatographie über Silica- Gel (n-Hexan/CH₂Cl₂ 9:1).
Ausbeute: 15,1 g (63 %, 41 mmol).

### V.6. rac-Dimethylsilylbis{1-(2-ethyl-4-methylindenyl)}zirkondichiorid (E)

Zu 3,57 (9,58 mmol) Me₂Si(2-Et-4-Me-lnd)₂ in 50 ml THF tropfte man bei Raumtemperatur 7,66 ml (19,16 mmol) einer 2,5 M BuLi-Lösung in n-Hexan zu und erwärmte noch 3 h auf 50°C. Es wurde zur Trockene eingedampft, in n-Pentan suspendiert, filtriert und getrocknet. 2,23 (9,58 mmol) ZrCl₄ wurden in 150 ml CH₂Cl₂ suspendiert und auf -78°C gekühlt. Das Dilithio-Salz wurde zugegeben, 3 h bei -20°C gerührt, und über Nacht auf Raumtemperatur kommen lassen. Es wurde filtriert und das Lösungsmittel entfernt. Kristallisation aus Toluol/n-Hexan (25:1) ergab 0,18 g orange Kristalle (meso/rac 5:1). Die Mutterlauge wurde auf 1/4 ihres Volumens eingeengt und der Kristallisation bei -38°C überlassen, man erhielt weitere 0,1 g des Komplexgemisches. Die Mutterlauge wurde zur Trockene eingedampft, der Rückstand in n-Hexan suspendiert, filtriert und getrocknet. Man erhielt die reine rac-Form E als orangefarbenes Pulver.

### VI. Metallocen F: rac-Dimethylsilylbis{1-(2,4-dimethylindenyl)} zirkondichlorid

### VI.1. (±)-2-Methyl-3-hydroxy-3-(2-tolyl)-propionsäuremethylester (f1)

42 g (645 mmol) Zn in 150 ml Toluol und 50 ml Et₂O wurden auf 80-85°C erhitzt, ein Gemisch aus 51,6 g (430 mmol) 2-Tolyl-aldehyd und 62 ml (557 mmol) 2-Brommethylmalonester zugetropft. Nach 5 % Zugabe wurde die Heizung entfernt und ein I₂-Kristall zugegeben. Nach heftigem Schäumen wurde dann der Rest bei 80-85°C innerhalb von 80 min zugetropft, 2 h bei 85°C gerührt und über Nacht stehen gelassen.
200 g Eis/30 ml H₂SO₄ wurden vorgelegt und der Ansatz zugegossen. Nach Ausethern, Waschen der organischen Phase mit NaHCO₃- und NaCl-Lösung wurde getrocknet und destilliert (101°C/1 Torr).
Ausbeute: 86 g (96 %)

### VI.2. (±)-2-Methyl-3-(2-tolyl)-propionsäuremethylester (f2)

Zu 86 g (413 mmol) β-Hydroxyester f1 in 800 ml CH₂Cl₂ wurden 132 ml (826 mmol) HSiEt₃ zugegeben. Bei -5 → -10°C wurden 102 ml (826 mmol) BF₃-Ether in 3 Portionen innerhalb 5-10 min zugegeben. Nach 20 h bei Raumtemperatur wurde aufgearbeitet. Nach Hydrolyse mit 220 ml NaHCO₃ (pH 3) wurde ausgeethert, die organische Phase abgetrennt, mit NaCl-Lösung gewaschen, getrocknet und destilliert (120°C/1 Torr).
Ausbeute: 58,9 g (74,1 %)

### VI.3. (±)-2-Methyl-3-(2-tolyl)-propionsäure (f3)

38,45 g (200 mmol) Ester f2, 850 ml 5 %ige NaOH und 850 ml MeOH wurden 4,5 h refluxiert, das MeOH abdestilliert , angesäuert, der Etherextrakt mit MgSO₄ getrocknet und destilliert (107-109°C/Hochvakuum).
Ausbeute: 31,8 g (89 %)

### VI.4. (±)-2-Methyl-3-(2-tolyl)-propionsäurechlorid (f4)

16,04 g (90 mmol) Säure f3 wurden mit 19,6 g (270 mmol) SOCl₂ langsam auf 80°C erhitzt und bis zum Ende der Gasentwicklung bei dieser Temperatur gehalten. Zum Entfernen des SOCl₂ wurde mehrmals mit Toluol eingedampft.
Ausbeute: 17,7 g (roh)

### VI.5. (±)-2,4-Dimethylindanon (f5)

Zu 17,7 g (90 mmol) Säurechlorid f4 in 50 ml Toluol wurden 36 g (270 mmol) AlCl₃ innerhalb 20 min gegeben und 4 h bei 80°C gerührt. Es wurde auf Eis/HCl gegossen, mit Toluol extrahiert, mit H₂O, NaHCO₃- und NaCl-Lösung gewaschen, getrocknet und destilliert (109°C/1 Torr) oder chromatographiert (n-Hexan/Ethylacetat 6:1, r_{F} = 0,44).
Ausbeute: 13,75 g (95,4 %).

Die Schritte VI.1. bis VI.5. wurden analog zu Synth. Comm., 20 (1990) 1387-97 durchgeführt.

### VI.6. (±)-2,4-Dimethylindanol (f6)

Zu 10,03 g (62,6 mmol) Keton f5 in 150 ml THF/MeOH 2:1 wurden bei 0°C 3,55 g (93,9 mmol) NaBH₄ portionsweise innerhalb 1 h gegeben. 2 h wurde bei 0°C, dann über Nacht bei Raumtemperatur gerührt. Es wurde auf Eis/HCl gegeben, pH 1 eingestellt, evtl. an der Phasengrenze ausgefallene Borsäure(?) abfiltriert, mit Et₂O extrahiert, mit NaHCO₃- und NaCl-Lösung gewaschen und an der Ölpumpe getrocknet.
Ausbeute: 10,24 g

### VI.7. 2,4-Dimethylinden (f7)

10,24 g (62 mmol) Indanol f6 wurden in Toluol gelöst und 20 mg p-Tolylsulfonsäurehydrat zugegeben. 2,5 h auf dem Dampfbad stehen gelassen, etwas Wasser zugefügt, die organische Phase eingedampft und destilliert (133°C/10 Torr).
Ausbeute: 8,63 g (95 %).

### VI.8. (±)-Dimethylsilyl-bis(2,4-dimethylinden) (f8)

Zu 8,63 g (59,8 mmol) Ligand f7 in 100 ml Et₂O wurden 37,4 ml 1,6 M (59,8 mmol) n-BuLi-n-Hexan-Lösung zugetropft und mehrere Stunden bei 40°C gerührt. Das Li-Salz wurde langsam zu 3,86 ml (29,9 mmol) Me₂SiCl₂ in 30 ml Et₂O getropft und 2 h gerührt. Nach Filtration wurde eingedampft und chromatographiert (n-Hexan/CH₂Cl₂ 9:1 r_{F} = 0,29). Die Produktfraktionen wurde vereinigt und aus MeOH umkristallisiert.
Ausbeute: 1,25 g (12%)

### VI.9. rac-Dimethylsilylbis{1-(2,4-dimethylindenyl)}zirkondichlorid (F)

1,25 g (3,63 mmol) Chelatligand f8 wurden in 20 ml THF gelöst, 2,9 ml 2,5 M (7,26 mmol) n-BuLi-n-Hexan-Lösung zugetropft und 2 h bei -40°C gerührt; bis zur Beendigung der Butanfreisetzung.

0,85 g (3,63 mmol) ZrCl₄ wurden in 30 ml CH₂Cl₂ suspendiert. Nach Zugabe des Dilithiosalzes bei -78°C wurde langsam auf Raumtemperatur erwärmt und nach Stehen über Nacht filtriert. Das Filtrat wurde i.V. eingedampft. Man erhielt den Komplex als eine Mischung der racemischen mit der meso-Form im Verhältnis 1:1 (orangefarbenes Pulver). Die reine racemische Form konnte durch Umkristallisation aus Toluol gewonnen werden. Reinausbeute 15 %. ¹H-NMR des Racemats (CDCl₃): 6,8-7,5 (m,6,Arom.-H), 6,82 (s,2,β-H), 2,3 (s,6,CH₃), 2,1 (s,6,CH₃), 1,30 (s,6,Si-CH₃).

### VII. Metallocen G: rac-Dimethylsilylbis{1-(2-methyl-4-ethylindenyl)} zirkondimethyl

0,26 g Metallocen A in 40 cm³ Et₂O wurden bei -50°C tropfenweise mit 1,3 cm³ einer 1,6 M (2,08 mmol) etherischen MeLi-Lösung versetzt und 2 h bei -10°C gerührt. Nach Austausch des Lösemittels gegen n-Pentan wurde noch 1,5 h bei Raumtemperatur gerührt und der filtrierte Rückstand im Vakuum sublimiert. Es wurden 0,15 g Sublimat mit einer korrekten Elementaranalyse erhalten.

### VIII. Umsetzung von Metallocen G mit [Bu₃NH][B(C₆H₅)₄]

0,15 g Metallocen G wurden bei 0°C zu 0,17 g [Bu₃NH][B(C₆H₅)₄] in 25 cm³ Toluol gegeben. Unter Rühren wurde auf 50°C erwärmt und die Mischung 10 min bei dieser Temperatur gerührt. Die tiefgefärbte Mischung wurde dann zur Trockne eingedampft. Für die Polymerisation wurde ein aliquoter Teil des Reaktionsgemisches verwendet (Bu = Butyl).
Abkürzungen: Me= Methyl, Et= Ethyl, Bu= Butyl, Ind= Indenyl.

## Patentansprüche

1. Inden der Formel (I) oder (II) 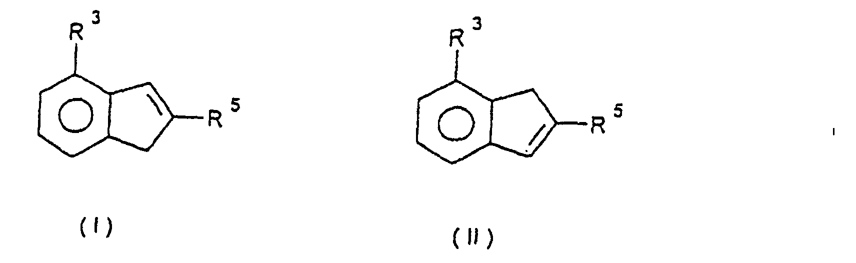 worin
R³ ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰ oder -PR₂¹⁰-Rest bedeutet, worin R¹⁰ ein Halogenatom, eine C₁-C₁₀-Alkylgruppe oder eine C₆-C₁₀-Arylgruppe ist, und
R⁵ die für R³ genannte Bedeutung hat.

2. Verfahren zur Herstellung eines Indens der Formel (I) oder (II) gemäß Anspruch 1 wobei
a) ein in 2-Position substituiertes Benzylhalogenid durch Umsetzen mit einem entsprechend substituierten Malonsäurediester in den disubstituierten Malonsäurediester umgewandelt wird,
b) der Malonsäurediester verseift und decarboxyliert wird,
c) die erhaltene Carbonsäure in das Carbonsäurechlorid überführt wird und der Ringschluß zum 1-Indanon durchgeführt wird, welches anschließend
d) reduziert wird und unter Wasserabspaltung das inden liefert.

3. Verwendung eines Indens gemäß Anspruch 1 zur Herstellung eines Metallocens der Formel (III) 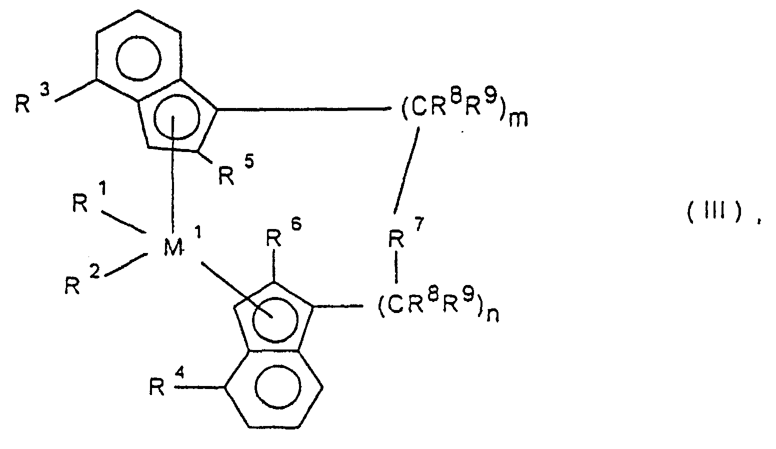 worin
M¹ ein Metall der Gruppe IVb, Vb oder Vlb des Periodensystems ist,
R¹ und R² gleich oder verschieden sind und Wasserstoff, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
R³ und R⁴ gleich oder verschieden sind und ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰, SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁵ und R⁶ gleich oder verschieden sind und die für R³ und R⁴ genannte Bedeutung haben,
R⁷ 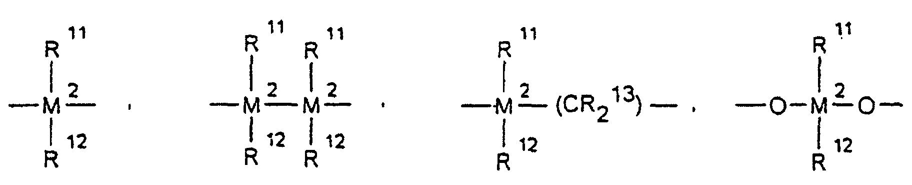 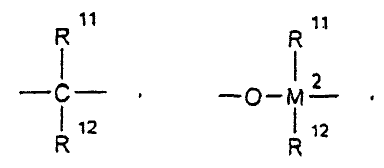
=BR¹¹, =AlR¹¹, -Ge-, -Sn-, -O-, -S-, =SO, =SO₂, =NR¹¹, =CO, =PR¹¹ oder =P(O)R¹¹ ist,
wobei
R¹¹, R¹² und R¹³ gleich oder verschieden sind und ein Wasserstoffatom, ein Halogenalom, eine C₁-C₁₀-Alkylgruppe, C₁-C₁₀-Fluoralkylgruppe, eine C₆-C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀-Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden, oder R¹¹ oder R¹² mit R⁸ oder R⁹ jeweils zusammen mit den sie verbindenden Atomen einen Ring bilden,
M² Silizium, Germanium oder Zinn ist,
R⁸ und R⁹ gleich oder verschieden sind und die für R¹¹ genannte Bedeutung haben und
m und n gleich oder verschieden sind und null, 1 oder 2 sind, wobei m plus n null, 1 oder 2 ist.

4. 1-lndanon der Formel (IV) 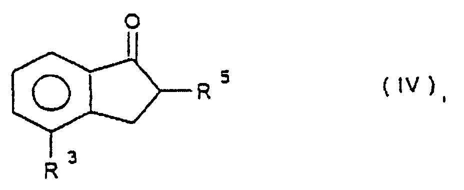 worin
R³ ein Halogenatom, eine C₁-C₁₀-Alkylgruppe, die halogeniert sein kann, eine C₆-C₁₀-Arylgruppe, einen -NR₂¹⁰, -SR¹⁰, -OSiR₃¹⁰ oder -PR₂¹⁰-Rest bedeutet, worin R¹⁰ ein Halogenatom, eine C₁-C₁₀-Alkylgruppe oder eine C₆-C₁₀-Arylgruppe ist, und
R⁵ die für R³ genannte Bedeutung hat.

5. Verwendung eines 1-Indanons gemäß Anspruch 4 zur Herstellung eines Indens wie in Anspruch 1 definiert.

6. Verfahren zur Herstellung eines Indens der Formel (I) oder (II) gemäß Anspruch 1,
wobei
a) ein Ketoaldehyd mit einem Cyclopentadien in einem inerten Lösungsmittel in Gegenwart einer Base zu einem in 4-Position monosubstituierten Inden umgesetzt wird,
b) das in 4-Position monosubstituierte Inden zu einem in 4-Position monosubstituierten 2-Indanon umgewandelt wird,
c) in welches durch Grignard-Reaktion der Rest R⁵ eingeführt wird, und
d) die anschließende Wasserabspaltung zu einem Inden der Formel (I) oder (II) führt.

## Claims

1. An indene of the formula (I) or (II) 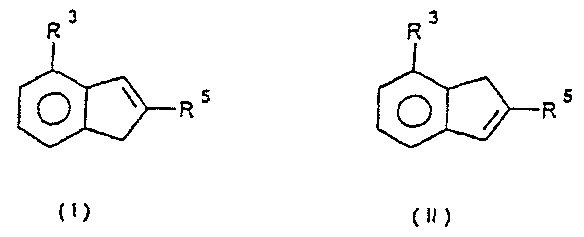 where
R³ is a halogen atom, a C₁-C₁₀-alkyl group which may be halogenated, a C₆-C₁₀-aryl group or an -NR₂¹⁰, -SR¹⁰, -OSiR₃¹⁰ or -PR₂¹⁰ radical, where R¹⁰ is a halogen atom, a C₁-C₁₀-alkyl group or a C₆-C₁₀-aryl group, and
R⁵ is as defined for R³.

2. A process for preparing an indene of the formula (I) or (II) as claimed in claim 1, which comprises
a) converting a benzyl halide substituted in the 2 position into the disubstituted malonic diester by reaction with an appropriately substituted malonic diester,
b) hydrolyzing and decarboxylating the malonic diester,
c) converting the resulting carboxylic acid into the carboxylic chloride and carrying out ring closure to form the 1-indanone, and subsequently
d) reducing this 1-indanone with elimination of water to give the indene.

3. The use of an indene as claimed in claim 1 for preparing a metallocene of the formula (III) 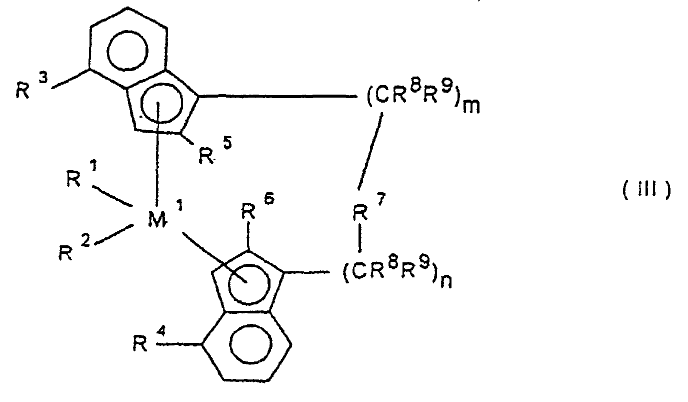 where
M¹ is a metal of group IVb, Vb or VIb of the Periodic Table,
R¹ and R² are identical or different and are each hydrogen, a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₇-C₄₀-alkylaryl group, a C₈-C₄₀-arylalkenyl group or a halogen atom,
R³ and R⁴ are identical or different and are each a halogen atom, a C₁-C₁₀-alkyl group which may be halogenated, a C₆-C₁₀-aryl group or an -NR₂¹⁰, -SR¹⁰, -OSiR₃¹⁰, SiR₃¹⁰ or -PR₂¹⁰ radical, where R¹⁰ is a halogen atom, a C₁-C₁₀-alkyl group or a C₆-C₁₀-aryl group,
R⁵ and R⁶ are identical or different and are as defined for R³ and R⁴,
R⁷ is 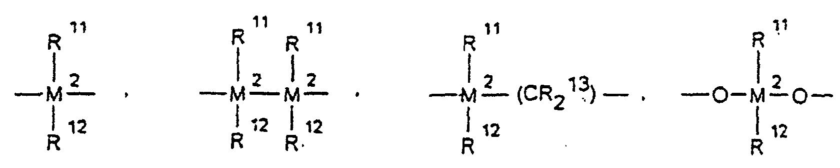 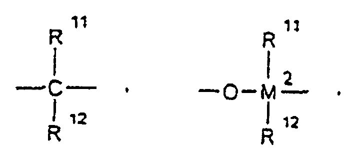
=BR¹¹, =AIR¹¹, -Ge-, -Sn-, -O-, -S-, =SO, =SO₂, =NR¹¹, =CO, =PR¹¹ or =P(O)R¹¹,
where
R¹¹, R¹² and R¹³ are identical or different and are each a hydrogen atom, a halogen atom, a C₁-C₁₀-alkyl group, a C₁-C₁₀-fluoroalkyl group, a C₆-C₁₀-aryl group, a C₆-C₁₀-fluoroaryl group, a C₁-C₁₀-alkoxy group, a C₂-C₁₀-alkenyl group, a C₇-C₄₀-arylalkyl group, a C₈-C₄₀-arylalkenyl group, a C₇-C₄₀-alkylaryl group or R¹¹ and R¹² or R¹¹ and R¹³ in each case together with the atoms connecting them form a ring, or R¹¹ or R¹² together with R⁸ or R⁹ and, in each case, the atoms connecting them form a ring,
M² is silicon, germanium or tin,
R⁸ and R⁹ are identical or different and are as defined for R¹¹ and
m and n are identical or different and are each zero, 1 or 2, where m plus n is zero, 1 or 2.

4. A 1-indanone of the formula (IV) 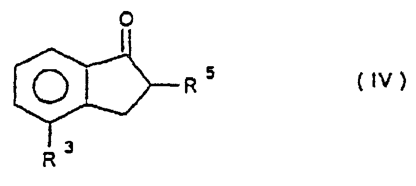 where
R³ is a halogen atom, a C₁-C₁₀-alkyl group which may be halogenated, a C₆-C₁₀-aryl group or an -NR₂¹⁰, -SR¹⁰, -OSiR₃¹⁰ or -PR₂¹⁰ radical, where R¹⁰ is a halogen atom, a C₁-C₁₀-alkyl group or a C₆-C₁₀-aryl group, and
R⁵ is as defined for R³.

5. The use of a 1-indanone as claimed in claim 4 for preparing an indene as defined in claim 1.

6. A process for preparing an indene of the formula (I) or (II) as claimed in claim 1, which comprises
a) reacting a ketoaldehyde with a cyclopentadiene in an inert solvent in the presence of a base to form an indene which is monosubstituted in the 4 position,
b) converting the indene which is monosubstituted in the 4 position into a 2-indanone which is monosubstituted in the 4 position,
c) introducing the radical R⁵ into the monosubstituted 2-indanone by means of a Grignard reaction, and
d) subsequently eliminating water from the product from c) to give an indene of the formula (I) or (II).

## Revendications

1. Indènes de formule (I) ou (II) 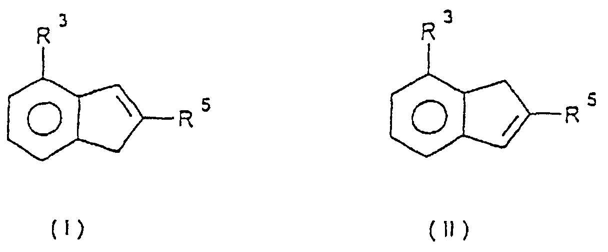 où
R³ désigne un atome d'halogène, un groupe alkyle en C₁-C₁₀, qui peut être halogéné, un groupe aryle en C₆-C₁₀, ou un reste -NR₂¹⁰, -SR¹⁰, -OSiR₂¹⁰ ou -PR₂¹⁰, tandis que R¹⁰ est un atome d'halogène, un groupe alkyle en C₁-C₁₀ ou un groupe aryle en C₆-C₁₀, et
R⁵ a la signification indiquée pour R³.

2. Procédé pour la préparation d'un indène de formule (I) ou (II) selon la revendication 1, dans lequel
a) un halogénure de benzyle substitué sur la position 2 est transformé par réaction avec un diester d'acide malonique substitué de façon correspondante en le diester d'acide malonique disubstitué,
b) le diester d'acide malonique est saponifié et décarboxylé,
c) l'acide carboxylique obtenu est converti en le chlorure d'acide carboxylique, qui ensuite
d) est réduit et donne l'indène par élimination d'eau.

3. Utilisation d'un indène selon la revendication 1 pour la préparation d'un métallocène de formule (III) 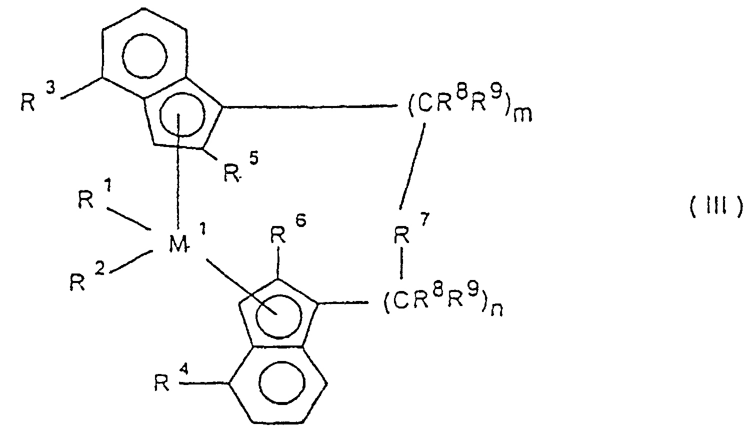 où
M¹ est un métal du groupe IVb, Vb ou VIb de la classification périodique des éléments,
R¹ et R² sont identiques ou différents et représentent l'hydrogène, un groupe alkyle en C₁-C₁₀, un groupe alcoxy en C₁-C₁₀, un groupe aryle en C₆-C₁₀, un groupe aryloxy en C₆-C₁₀, un groupe alcényle en C₂-C₁₀, un groupe arylalkyle en C₇-C₄₀, un groupe alkylaryle en C₇-C₄₀, un groupe arylalcényle en C₈-C₄₀ ou un atome d'halogène,
R³ et R⁴ sont identiques ou différents et représentent un atome d'halogène, un groupe alkyle en C₁-C₁₀, qui peut être halogéné, un groupe aryle en C₆-C₁₀, un reste -NR₂¹⁰, -SR¹⁰, -OSiR₃¹⁰, SiR₃¹⁰ ou -PR₂¹⁰, tandis que R¹⁰ est un atome d'halogène, un groupe alkyle en C₁-C₁₀ ou un groupe aryle en C₆-C₁₀,
R⁵ et R⁶ sont identiques ou différents et ont la signification indiquée pour R³ et R⁴,
R⁷ 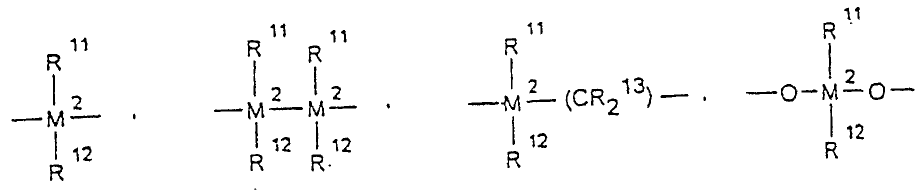 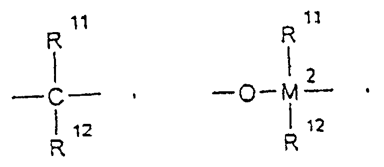
=BR¹¹, =AlR¹¹, -Ge-, -Sn-, -O-, -S-, =SO, =SO₂, =NR¹¹, =CO, =PR¹¹ ou =P(O)R¹¹,
tandis que
R¹¹, R¹² et R¹³ sont identiques ou différents et représentent un atome d'hydrogène, un atome d'halogène, un groupe alkyle en C₁-C₁₀, un groupe fluoroalkyle en C₁-C₁₀, un groupe aryle en C₆-C₁₀, un groupe fluoroaryle en C₆-C₁₀, un groupe alcoxy en C₁-C₁₀, un groupe alcényle en C₂-C₁₀, un groupe arylalkyle en C₇-C₄₀, ou R¹¹ et R¹² ou R¹¹ et R¹³ forment à chaque fois un cycle avec les atomes qui les relient, ou R¹¹ ou R¹² forment un cycle avec R⁸ ou R⁹ à chaque fois conjointement avec les atomes les reliant,
M² est le silicium, le germanium ou l'étain,
R⁸ et R⁹ sont identiques ou différentes et ont la signification indiquée pour R¹¹ et
m et n sont identiques ou différents et valent zéro, 1 ou 2, tandis que m plus n est zéro, 1 ou 2.

4. 1-indanone de formule (IV) 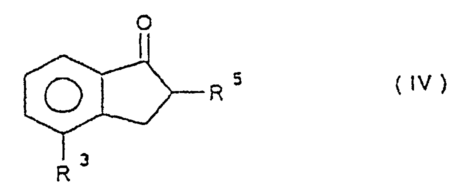 où
R³ est un atome d'halogène, un groupe alkyle en C₁-C₁₀, qui peut être halogéné, un groupe aryle en C₆-C₁₀, un reste -NR₂¹⁰, -SR¹⁰, -OSiR₃¹⁰ ou -PR₂¹⁰, tandis que R¹⁰ est un atome d'halogène, un groupe alkyle en C₁-C₁₀ ou un groupe aryle en C₆-C₁₀, et
R⁵ a la signification indiquée pour R³.

5. Utilisation d'une 1-indanone selon la revendication 4 pour la préparation d'un indène tel que défini dans la revendication 1.

6. Procédé pour la préparation d'un indène de formule (I) ou (II) selon la revendication 1, dans lequel
a) on fait réagir un cétoaldéhyde avec un cyclopentadiène dans un solvant inerte en présence d'une base pour former un indène monosubstitué sur la position 4,
b) l'indène monosubstitué sur la position 4 est transformé en une 2-indanone monosubstituée sur la position 4,
c) dans laquelle on introduit par réaction de Grignard le reste R⁵, et
d) l'élimination subséquente d'eau conduit à un indène de formule (I) ou (II).
